(19) Europäisches Patentamt

European Patent Office

Office européen des brevets

(11) Publication number: **0 405 467 A2**

# EUROPEAN PATENT APPLICATION

(21) Application number: 90112163.2

(22) Date of filing: 26.06.90

(51) Int. Cl.⁵: **G01N 33/68**, //C12N15/26

(30) Priority: 27.06.89 US 371898

(43) Date of publication of application:
**02.01.91 Bulletin 91/01**

(84) Designated Contracting States:
**CH DE FR GB IT LI NL**

(71) Applicant: **F. HOFFMANN-LA ROCHE AG**
**Postfach 3255**
**CH-4002 Basel(CH)**

(72) Inventor: **Chizzonite, Richard A.**
**104 Richards Road**
**South Kent, Connecticut 06785(US)**

(74) Representative: **Lederer, Franz, Dr. et al**
**Patentanwalt Dr. Franz Lederer**
**Lucile-Grahn-Strasse 22**
**D-8000 München 80(DE)**

(54) A process for the determination of interleukins.

㊼ The invention provides a novel method for the immunoassay of therapeutic unglycosylated IL-2 which are unaffected by the presence of endogenous glycosylated IL-2.

# A PROCESS FOR THE DETERMINATION OF INTERLEUKINS

Within the last decade, it has become clear that immunocompetent cells produce both antigen-specific and nonspecific molecules which enable them to communicate with other immunocompetent cells in the generation and control of the immune response [Waksman, in Lymphokine Reports, Vol. I, 1980. Pick (Ed.), Academic Press, New York, 1; Germain, in Lymphokine Reports, Vol. I, 1980 Pick (Ed.), Academic Press, New York, 7]. When such molecules are secreted by B- or T-lymphocytes, they are termed lymphokines; when produced by macrophages or monocytes, they are called monokines. These protein and glycoprotein molecules, which are secreted by cells and which act upon other cells in close proximity to them, have been classified as cytokines. This type of local cell regulation by protein and peptide molecules has been termed autocrine to distinguish this type of cellular regulation from the endocrine system, where mediators are carried by the blood and act on target tissues at distant sites.

Interleukin-2 (IL-2) is one of a number of lymphokines which play a critical role in the regulation of immune responses. It was first identified as a discrete entity by Morgan, Ruscetti, and Gallo [Science 193 :1007 (1976)]. Previously known as T-cell growth factor (TCGF), IL-2 is secreted by T-lymphocytes after exposure to mitogens or antigens. It is this second signal which further stimulates proliferation of T-lymphocytes. This glycoprotein molecule is intimately involved in the induction and sustenance of virtually all immune responses in which T-cells play a role [Farrar et al ., J. Immunol. Rev. 63 :129 (1983)]. IL-2 is believed to provide a universal signal for the proliferation of mature T-cells through its binding to specific cell-surface receptors. IL-2 has been shown to induce antigen-specific cytotoxic T-lymphocytes, natural killer cells, or lymphokine-activated killer cells, all of which are implicated as effector cells in surveillance against malignancy, and to reconstitute functional T-cell responses and to assist in the immune T-cell- and chemotherapy-induced elimination of established murine syngeneic leukemias [Smith, Immunol. Rev. 51 :337 (1980); Robb et al ., J. EXP. Med. 154 :1455 (1981); Altman et al ., Proc. Natl. Acad. Sci. USA 81 :2176 (1984)].

Abnormalities in IL-2 production and/or response are associated with several diseases. Such defects have been identified in humans with systemic lupus erythematosus, in aged individuals, in children with primary immunodeficiencies, in bone marrow transplant recipients undergoing a graft-versus-host reaction and in parasite-infected mice. IL-2 may be useful as an immunological response modifier in cancer patients [Key et al ., Immunol. Letters 6 :175 (1983)].

Systemic administration of autologous lymphokine-activated killer (LAK) cells and recombinantly derived IL-2 to 25 advanced metastatic cancer patients resulted in regression of the cancer in 11 of the 25 patients [Rosenberg et al ., N. Eng. J. Med. 313 :1485 (1985)].

The entire primary sequence of Interleukin-2 has been described by Taniguchi et al . [Nature 302 :305 (1983)]. IL-2 contains 133 amino acids and has the following sequence:

```
1                                          10
Ala-Pro-Thr-Ser-Ser-Ser-Thr-Lys-Lys-Thr-Gln-Leu-Gln-Leu-Glu-
          20                                         30
His-Leu-Leu-Leu-Asp-Leu-Gln-Met-Ile-Leu-Asn-Gly-Ile-Asn-Asn-
                              40
Tyr-Lys-Asn-Pro-Lys-Leu-Thr-Arg-Met-Leu-Thr-Phe-Lys-Phe-Tyr-
              50                                    60
Met-Pro-Lys-Lys-Ala-Thr-Glu-Leu-Lys-His-Leu-Gln-Cys-Leu-Glu-
                              70
Glu-Glu-Leu-Lys-Pro-Leu-Glu-Glu-Val-Leu-Asn-Leu-Ala-Gln-Ser-
              80                                    90
Lys-Asn-Phe-His-Leu-Arg-Pro-Arg-Asp-Leu-Ile-Ser-Asn-Ile-Asn-
                             100
Val-Ile-Val-Leu-Glu-Leu-Lys-Gly-Ser-Glu-Thr-Thr-Phe-Met-Cys-
             110                                   120
Glu-Tyr-Ala-Asp-Glu-Thr-Ala-Thr-Ile-Val-Glu-Phe-Leu-Asn-Arg-
                             130        133
Trp-Ile-Thr-Phe-Cys-Gln-Ser-Ile-Ile-Ser-Thr-Leu-Thr.
```

The amino acids and the corresponding three-letter designations and single-letter designations are as follows:

| Amino Acid | 3-Letter | 1-Letter |
|---|---|---|
| Alanine | Ala | A |
| Arginine | Arg | R |
| Asparagine | Asn | N |
| Aspartic Acid | Asp | D |
| Cysteine | Cys | C |
| Glutamic Acid | Glu | E |
| Glutamine | Gln | Q |
| Glycine | Gly | G |
| Histidine | His | H |
| Homoserine | Hse | - |
| Isoleucine | Ile | I |
| Leucine | Leu | L |
| Lysine | Lys | K |
| Methionine | Met | M |
| Phenylalaine | Phe | F |
| Proline | Pro | P |
| Serine | Ser | S |
| Theronine | Thr | T |
| Tryptopan | Trp | W |
| Tyrosine | Tyr | Y |
| Valine | Val | V |

Although it may theoretically be possible to synthetically produce IL-2, this method is not yet feasible for the production of quantities of homogenous material. IL-2 is presently being produced recombinantly in fermentation culture. Monoclonal antibodies are useful analytical reagents for the large scale production and purification of IL-2.

Gillis et al . [J. Immunol. 126 :1978 (1981)] reported the production of a B cell hybridoma whose antibody product inhibited IL-2 activity. BALB/c female mice were immunized with IL-2 isolated from rat splenocytes. The spleens of the immunized mice were removed and single cell suspensions were produced. These spleen cells were fused with the BALB/c myeloma Sp2/0. A monoclonal B-cell hybridoma was isolated whose IgG product appeared to be directed against a determinant present on molecules of human, mouse and rat IL-2 (Altman et al ., supra ).

Stadler et al ., [J. Immunol. 128 :1620 (1982)] immunized BALB/c mice with partially purified human IL-2. The spleens of the immunized mice were harvested and the spleen cells were hybridized with plasmacytoma cells. Supernatants of the hybridoma cultures were screened for their ability to inhibit IL-2 proliferation of the CT6 cell line. Eight different lines were found to produce antibodies which inhibited the proliferation of the IL-2 dependent cell line in response to either crude or purified human IL-2 as well as rat and mouse IL-2. These anti-IL-2 antibodies did not inhibit the proliferation of human T-cell lines capable of producing IL-2.

Smith et al ., [J. Immunol. 131 :1808 (1983)] have described the preparation of three monoclonal antibodies to IL-2 which are identified as DMS-1, DMS-2 and DMS-3. DMS-1 and 2 reacted specifically with IL-2 as demonstrated by antibody concentration-dependent neutralization of IL-2 activity. DMS-3, although less effective in neutralizing IL-2, bound to IL-2 more avidly and functioned as an immunoabsorbent.

Altman et al ., [Proc. Natl. Acad. Sci. USA 81 :2176 (1984)] have described the preparation of polyclonal antibodies to human IL-2, using as immunogens eight synthetic peptides derived from the predicted amino acid sequence of IL-2. Each peptide consisted of 13-15 amino acids and was derived from the IL-2 sequence data published by Taniguchi et al ., supra . Antibodies to four of the eight peptides were found to bind to IL-2. An affinity-purified antibody to one of the IL-2 peptides specifically stained the cytoplasm of phytohemagglutinin-stimulated human peripheral blood lymphocytes. None of these antibodies demonstrated neutralization of IL-2 activity.

Robb et al ., [Proc. Natl. Acad. Sci. USA 80 :5990 (1983)] have reported the preparation of a monoclonal antibody designated IH II-IA5 which specifically reacted with the carbohydrate residue at amino acid 3 of IL-2. This antibody was used for affinity purification of glycosylated IL-2 but did not inhibit the biological activity of IL-2.

Monoclonal antibodies to IL-2 provide powerful analytical and diagnostic tools for the study of IL-2 biological activity as well as the immunopurification of IL-2, the development of IL-2 immunoassays, the identification of the active site of the IL-2 molecule and an assay for in vivo studies exploring the physiological role of IL-2 in the immune response. Although monoclonal antibodies to IL-2 are known, few are available which may be routinely utilized as affinity reagents for the purification of IL-2 and as reagents in a sensitive two-site immunoassay for IL-2.

The present invention provides monoclonal antibodies against IL-2 which have utility in the investigation of the regions of IL-2 responsible for biological activity and as affinity reagents for the purification of human and mouse IL-2 as reagents for a sensitive two-site immunoassay. The invention further provides methods for the use of the novel monoclonal antibodies of the invention as an affinity matrix to purify IL-2 and for the use of the monoclonal antibodies of the invention in a two-site immunoassay for quantifying IL-2 in a biological sample, e.g. , serum or plasma.

Some of the monoclonal antibodies of the invention are characterized by an ability to bind to and neutralize the biological activity of unglycosylated IL-2 and an inability to bind to glycosylated IL-2.

All references cited herein are hereby incorporated in their entirety by reference.

The invention is directed to monoclonal antibodies specific to native IL-2, recombinant IL-2, and IL-2 synthetic peptides. The invention is further directed to three groups of monoclonal antibodies having the following characteristics;

Group 1. Monoclonal antibodies which inhibit IL-2 function to greater than 50% in both the IL-2 Bioassay and the Receptor Binding Assay and which bind (i.e., recognizes the epitope) between amino acids 1-12, 9-19, 41-55 or 21-123 on the native or recombinant human IL-2 protein.

Group 2. Monoclonal antibodies which have high affinity for native or recombinant human IL-2 as measured by IL-2 RIA assay (radioimmunoassay), which do not inhibit IL-2 function when measured by the IL-2 Bioassay and Receptor Binding Assay and which bind between amino acids 40-70, 42-56, 21-123, 78-87, 52-70, 66-87, 107-121 or 71-87 on the native or recombinant human IL-2 protein.

Group 3. Monoclonal antibodies which inhibit native or recombinant mouse and human IL-2 in the IL-2 Bioassay and the Receptor Binding Assay and which bind between amino acids 41-55 on the IL-2 protein.

The monoclonal antibodies (mAbs) of Group 1 inhibit IL-2 function to greater than 50% in the IL-2 Neutralization Assay and in the Receptor Binding Assay. The mAbs of Group I recognize or bind to an epitope located at amino acids 1-12, 9-19, 41-55 or 21-123 on the native or recombinant IL-2 protein. These mAbs can be used to identify two separate amino acid sequences which are necessary for the bioactivity of IL-2; i.e., sequences 9-19 and 41-55.

Some of the monoclonal antibodies of Group 1 of this invention can specifically bind to and neutralize the growth-promoting biological activity of unglycosylated human IL-2 but do not bind to glycosylated human IL-2. These antibodies, which will hereinafter be referred to as "carbohydrate-blocked" antibodies, bind to the threonine residue at position 3, near the amino-terminus of IL-2,

Robb et al . [Proc. Natl. Acad. Sci. USA 81 :6486 (1984)] have shown that natural IL-2 made by induced JURKAT cells is composed of three major species having the normal amino acid sequence (Taniguchi et al ., supra ) which differ in molecular size and in the degree of glycosylation. One IL-2 species made by such cells has a molecular weight of about 15,600 daltons and is completely unglycosylated. The other two major species are glycosylated in different ways at position 3. The carbohydrate-blocked antibodies of this invention cannot bind to the glycosylated IL-2 species because the carbohydrate attached to the threonine at position 3 inhibits binding at that point.

Because of the unique ability of the carbohydrate-blocked monoclonal antibodies of the invention to bind to unglycosylated IL-2 but not to glycosylated IL-2, they can be used in an immunoassay to more accurately monitor blood levels of unglycosylated recombinant IL-2 administered therapeutically to patients. Endogeneous levels of natural, glycosylated IL-2 will of course not be recognized by these antibodies and thus will not affect the results obtained in such immunoassay using these antibodies.

As an example of a carbohydrate-blocked mAb, antibody 5B1 was prepared and characterized as described below. It must be stressed, however, that other carbohydrate-blocked mAbs can reauily be prepared using analogous procedures and routine experimentation. Hybridomas are made using unglycosylated IL-2 as an antigen and screened as described below using a small peptides the amino acid sequence of which corresponds to a subsequence of human IL-2 (comprising from about 10 to about 40 amino acids) and contains the 3-position threonine. Antibodies from hybridoma clones that are positive by such screening are then tested in an immunoassay, using glycosylated and unglycosylated IL-2 to identify those which bind to the unglycosylated IL-2 only.

The monoclonal antibodies of Group 2 do not inhibit IL-2 function. Group 2 monoclonal antibodies of the invention have a high affinity ($10^7$-$10^9$ M$^{-1}$) for IL-2 when measured by Radio Immunoassay and are thus useful as reagents for affinity chromatography, to purify or isolate human IL-2 and also for antibodies in the Two Site Immunoassay. Group 2 mAbs identify amino acid sequences which are not necessary for the bioactivity of IL-2.

The monoclonals of Group 3 inhibit both mouse IL-2 and human IL-2 function in the IL-2 Neutralization Assay and Receptor Binding Assay. The mAbs of Group 3 are particularly useful as modoel antiagonists of IL-2 action in both in vitro and in vivo assays. Furthermore, mAbs in this group are particularly suited for incorporation into an affinity matrix for the purification of human or mouse IL-2 and especially for the large scale production of mouse IL-2 which is needed for pre-clinical studies.

The term Enzyme Immunoassay ("EIA") refers to the following assay. Hybridoma supernatents are reacted with either r-IL-2 or IL-2 synthetic peptides immobilized on a solid substrate, e.g. , a microtiter plate. Following sequential incubations with goat anti-mouse IgG coupled to peroxidase and o-phenylenediamine, the amount of antibody bound to r-IL-2 or IL-2 peptide is determined by reading the optical density (OD 488 nm) of each well. A positive result is scored if the specific binding is greater than 10x background value.

The term "Neutralization Assay' refers to a conventional T-cell growth assay as described by Gillis et al . [J. Immunol. 120 :2027 (1978)]. Minor modifications to the standard procedure are within the skill of the art and may be appropriate depending upon individual requirements. Briefly, an IL-2 dependent cloned murine cytotoxic T-cell line is incubated in the presence of IL-2 and the monoclonal antibody to be tested. If the monoclonal antibody binds to a "neutralizing" epitope on the IL-2 protein, IL-2 will not be available tor uptake by the T-cell line; that is, the monoclonal antibody "neutralizes" the IL-2 activity. If the mAb does not bind to a neutralizing epitope on the IL-2 protein, IL-2 will be available for uptake by the T-cell line.

The term "Binding Assay" refers generally to a procedure developed by Robb et al . [J. Exp. Med. 154 :1455 (1981)]. As used herein the term specifically refers to an assay which measures the ability of the monoclonal antibody to inhibit the binding of radiolabelled IL-2 ($^{125}$I-IL-2) to receptor sites on target cells.

The term "epitope" (sometimes referred to as an "antigenic determinant") is an area of the IL-2 molecule to which a monoclonal antibody of the invention binds. This "area of the IL-2 molecule" is the amino acid sequence recognized by the mAb. According to convention, the amino acids in the IL-2 protein

5

are numbered starting from the N-terminal amino acid; thus the amino acid segment 1-12 is the first twelve amino acids of the IL-2 protein.

The term "Radio Immunoassay" (RIA) refers to an assay which measures the ability of the monoclonal antibodies of the invention to bind to $^{125}$I labelled IL-2. The monoclonal antibodies of the invention are incubated with goat anti-mouse IgG coupled to a solid substrate (agarose beads). The beads are pelleted by centrifugation and the pellet is incubated with $^{125}$I-labelled IL-2. After pelleting and washing to remove unbound $^{125}$I-IL-2, the affinity of the mAbs for the labelled IL-2 is determined either by the method of Muller [Methods in Enzymology 92 :589 (1983)] or by the method of Scatchard [Ann. N.Y. Acad. Sci. 51 :660 (1949)]. The radioactivity is counted using a gamma counter.

Peptides were derived from the published sequence of the amino acid sequence of human IL-2 [Robb et al ., [Proc. Natl. Acad. Sci. USA 81 :6486 (1980)]. Synthetic peptides were prepared by the Merriefield solid-phase method [Barany et al ., in The Peptides: Analysis, Synthesis, Biology Vol. 2, 1980, Gross et al . (Eds.), Academic press, New York, pp. 1-255: Tam et a l., J. Am. Chem. Soc. 105 :6442 (1983)]. The composition of all peptides was confirmed by amino acid analysis. The amino acid sequences of IL-2 and the synthetic peptides used to probe the hybridoma supernatants are shown below.

```
           1                                    10                                      20
H-Ala-Pro-Thr-Ser-Ser-Ser-Thr-Lys-Lys-Thr-Gln-Leu-Gln-Leu-Glu-His-Leu-Leu-Leu-Asp-
                                                30                                      40
    Leu-Gln-Met-Ile-Leu-Asn-Gly-Ile-Asn-Asn-Tyr-Lys-Asn-Pro-Lys-Leu-Thr-Arg-Met-Leu-
                                                50                                      60
    Thr-Phe-Lys-Phe-Tyr-Met-Pro-Lys-Lys-Ala-Thr-Glu-Leu-Lys-His-Leu-Gln-Cys-Leu-Glu-
                                                70                                      90
    Glu-Glu-Leu-Lys-Pro-Leu-Glu-Glu-Val-Leu-Asn-Leu-Ala-Gln-Ser-Lys-Asn-Phe-His-Leu-
                                                80
    Arg-Pro-Arg-Asp-Leu-Ile-Ser-Asn-Ile-Asn-Val-Ile-Val-Leu-Glu-Leu-Lys-Gly-Ser-Glu-
                                                110                                    120
    Thr-Thr-Phe-Met-Cys-Glu-Tyr-Ala-Asp-Glu-Thr-Ala-Thr-Ile-Val-Glu-Phe-Leu-Asn-Arg-
                                                130           133
    Trp-Ile-Thr-Phe-Cys-Gln-Ser-Ile-Ile-Ser-Thr-Leu-Thr-OH.
```

Contiguous Peptides :
1-12, 13-26, 27-41, 42-56, 57-70, 71-87, 88-105, 105-121, and 122-133.
Overlap peptides :
1-19, 9-26, 9-47, 10-47, 23-41, 30-70, 36-56, 40-70, 52-70, 66-87, 78-87, 84-105, 98-121, 107-121 and 116-133.

Recombinant IL-2 analogs were prepared as follows:

Defined amino acid changes were made by mutagenesis of a double-stranded DNA plasmid capable of expressing human IL-2 protein in E . coli . The procedure for site-specific mutagenesis utilizes synthetic oligonucleotides which code for amino acid changes and was performed as described by Morinaga et al . [Bio/Technology 2 :636 (1984)]. For each mutation, 100 ng of gapped and linear plasmid DNA were mixed with 1 pmole of phosphorylated synthetic oligonucleotide. The DNAs were denatured by heating, then allowed to anneal at room temperature. The single-stranded region of the heteroduplex molecules which formed was then filled in, incorporating the synthetic oligonucleotide. A sample of the DNA mixture was then transferred into E . coli strain MC1061. Bacterial colonies containing the mutated plasmid were identified by specific hybridization to the $^{32}$P-labelled synthetic oligonucleotide under conditions in which the wild-type IL-2 DNA did not hybridize. The mutated plasmid was induced to express the recombinant IL-2 protein analogue by growth of the host cells at 42° under standard conditions.

The analogs in which single amino acid changes were made are shown in Table 1. Note that two different analogs were prepared based upon substitutions at each of positions 17 and 45. Additionally, several deletion mutants were prepared in which amino acids 1-10, 1-20 and 124-133 were deleted from the native protein.

TABLE 1

Recombinant IL-2 Analogs

| Position of Amino Acid (AA) In Native IL-2 | 3 | 7 | 8 | 9 | 12 | 13 | 14 | 15 | 16 | 17 | 18 | 19 | 20 | 41 | 42 | 43 | 45 | 46 | 48 | 49 | 50 | 54 |
|---|---|---|---|---|---|---|---|---|---|---|---|---|---|---|---|---|---|---|---|---|---|---|
| AA in Native Protein | Thr | Thr | Lys | Lys | Leu | Gln | Leu | Glu | His | Leu | Leu | Leu | Asp | Thr | Phe | Lys | Tyr | Met | Lys | Lys | Ala | Lys |
| AA in Analog | Tyr | Val | Glu | Glu | Thr | Lys | Thr | Lys | Asp | Asn | Thr | Thr | Asn | Val | Tyr | Glu | Phe | Leu | Glu | Glu | Trp | Ala |
| | | | | | | | | | | Val | | | | | | | Arg | | | | | |

To achieve high titers of anti-IL-2 antibodies in the serum, it is important to use between about 25 to about 50 μg of immunogen, preferably about 50 μg of immunogen, at day 0 and 7 followed by a booster immunization of from about 50 μg to about 75 μg of immunogen at day 30 (preferably about 75 μg of immunogen) to obtain EIA and RIA titers of from about 1:0.5-10 X $10^5$ and about 1:1-2 x $10^3$ dilution respectively.

The following examples provide a more detailed description of the preparation and use of the monoclonal antibodies of the invention.

## Example 1

Immunizations

Eight female Balb/c mice (Charles River Laboratories, Wilmington, Mass.), 8-10 weeks of age, were immunized with 25 to 50 $\mu$g of recombinant human interleukin-2 (rIL-2) in complete Freund's adjuvant on days 0 and 7. The immunizations were at three sites: one intraperitoneal injection (i.p.) and two subcutaneous injections into the left and right inguinal region. On day 30 the mice were bled via the retro-orbital plexus and their sera were tested for anti-IL-2 antibodies in an enzyme immunoassay (EIA) and a radioimmunoassay (RIA). The mice were given an intraperitoneal booster immunization of 50-75 $\mu$g of rIL-2 on day 60 and were bled again on day 90. The sera were tested for anti-IL-2 antibodies and subsequently for ability to inhibit the bioactivity of IL-2. The inhibition of IL-2 bioactivity was evaluated by two methods: 1) neutralization of IL-2 dependent growth of mouse CTLL cells or human peripheral blood lymphocyte blasts, (Neutralization Assay) and 2) inhibition of binding of $^{125}$I-labelled IL-2 to target cells (Receptor Binding Assay). Both assays measure high affinity antibodies which bind epitopes on IL-2 that are necessary for IL-2 to bind the cellular receptor. The sera from day 90 demonstrated EIA and RIA titers of approximately 1:0.5-10 X $10^5$ and 1:1-2 x $10^3$ dilution, respectively, and also inhibited the bioactivity of IL-2. Forty five to 100 days after the second bleed, the mice were given an intravenous (i.v.) and an i.p. booster immunization on each of three successive days with 50 $\mu$g of rIL-2. On the fifth day, the mice were killed the spleens were removed for preparation of splenocytes.

## Example 2

Preparation of Hybridoma Cell Fusions

Two days before fusion, splenocyte feeder cells were prepared from naive mice in complete medium [IMDM + 10% fetal bovine serum, glutamine (2.0 mM), and 2-mercaptoethanol (100 $\mu$M) containing 100 $\mu$M hypoxanthine, 0.4 $\mu$M aminopterin, and 16 $\mu$M thymidine (HAT)]. Following a modification of the procedure of De St. Groth et al . [J. Immunol. Methods 35 :1 (1980)] spleen cells ($10^8$) were fused with $10^8$ PAI-O mouse myeloma cells growing in logarithmic phase. The cells were mixed, pelleted by centrifugation and resuspended under constant gentle agitation in 1.0 ml of 35% (v/v) polyethylene glycol in IMDM at 37° for 1 min. After incubation for 3 min. at 37°C, the cells were pelleted again by centrifugation and gently resuspended in 10 ml of IMDM + HAT. The cells were then diluted to 1 X $10^6$ cells per ml in complete medium + HAT and dispersed to 24-well microtiter plates (1 ml/well) containing 5 X $10^5$ feeder cells in 1 ml of complete medium. Hybridoma supernatants were assayed for anti-IL-2 antibodies by the IL-2 EIA and RIA as shown in Examples 3 and 4. Hybridomas were cloned by limiting dilution.

## Example 3

IL-2 EIA

Hybridoma culture supernatants (66 $\mu$l) were added to wells of microtiter plates (Falcon Probind or Costar EIA) which had been coated with either rIL-2 or with the IL-2 peptides described above (100 ng/well at 37°C overnight) and which contained 35 $\mu$l of 2% BSA in 25 mM sodium phosphate (pH 6.5), 1 M NaCl and 0.15% Tween 20. After incubation at room temperature for 2 hrs, the wells were washed with PBS (25 mM sodium phosphate (pH 7.4), 0.15 M NaCl) + 0.05% Tween 20. One hundred $\mu$l of goat anti-mouse IgG + IgM coupled to horseradish peroxidase [Boehringer-Mannheim Biochemicals, 1:1000 dilution in antibody buffer: 25 mM sodium phosphate (pH 6.5), 0.5 M NaCl + 0.05% Tween 20] was added to each well and incubated for 90 min. at room temperature. The wells were washed with PBS + 0.05% Tween 20, and incubated with o-phenylenediamine (0.4 mg/ml in 0.1 M citrate buffer (pH 4.5) + .012% hydrogen peroxide) for 30 min. at room temperature. The reaction was stopped by the addition of 50 $\mu$l of 2.5 M $H_2SO_4$ containing 50 mM sodium metabisulfite. The $OD_{488}$ of the substrate color was read on a Titertek Multiscan MC.

Example 4

IL-2 RIA

Hybridoma supernatants (0.1 - 0.5 ml) were incubated with 100 $\mu$l of a 50% suspension of goat anti-mouse IgG coupled to agarose beads (Sigma Chemical Co.) in 1% BSA in RIPA buffer (50 mM sodium phosphate (pH 7.5), 1% Triton X-100, 1% deoxycholate, 0.1% sodium dodecylsulfate, 0.15 M NaCl, 5 mM EDTA) for 2 hrs on a rotating mixer at room temperature. The beads were pelleted by centrifugation, washed 1x in RIPA buffer, and 250 $\mu$l of $^{125}$I labelled IL-2 (10-20 fmoles, 1 - 2 X $10^4$ cpm) in 1% BSA + RIPA buffer was added. After overnight incubation at 4°C on a rotating mixer, the beads were washed with RIPA buffer and counted in an LKB gamma counter.

Example 5

IL-2 Neutralization Assay

IL-2 bioactivity was determined by a microassay with the use of an IL-2 dependent cloned murine cytotoxic T-cell line (CTLL) as described by Gillis et al . Inhibition of IL-2 bioactivity was assayed by a modification of the IL-2 activity assay. Briefly, 25 $\mu$l of concentrated hybridoma supernatant (20X) was incubated with 25 $\mu$l of medium containing IL-2 (1 Unit/ml) for 1 hr at 37°C. This mixture was added to 50 $\mu$l of medium and CTLL cells in a microtiter plate and incubated overnight at 37°C. The next day the cultures were pulsed with $^3$H-thymidine (0.5 $\mu$Ci/50 $\mu$l/ well) and incubated for 5 hrs at 37°C. The incorporation of $^3$H-thymidine was determined by liquid scintillation counting, and the results were expressed as the percent inhibition of $^3$H-thymidine incorporation in the presence of antibody, compared to medium control.

Example 6

IL-2 Receptor Binding Assay

Inhibition of Binding of $^{125}$I-IL-2 to target cells by hybridoma antibodies was determined as described by Robb et al . [J. Exp. Med. 154 :1455 (1981)] with some modifications. Hybridoma supernatants (20x concentrate) were preincubated with $^{125}$I-IL-2 for 1 hr at 37°C. One hundred twenty $\mu$l aliquots ot murine CT6 or human peripheral blood lymphocyte blast cells (6 X $10^4$ cells) were added to give a final volume of 150 $\mu$l. After incubation for 20 min at 37°C, the cells were centrifuged for 90 sec at 4°C through a silicone oil mixture. Tube tips containing the cell pellets were excised and radioactivity was determined in a gamma counter. The results are expressed as percent inhibition of $^{125}$I-IL-2 bound in the presence of antibody compared to control values. Table 2 summarizes the results of the assays described in Examples 3-6. The amino acids in parenthesis, e.g. , "(Lys 8)" under the column labelled "epitope" are essential for binding to

## TABLE 2

### Comparison of the Functional Domains of IL-2 and Epitopes of Monoclonal Anti-IL-2 Antibodies

| MONOCLONAL ANTIBODY | | EPITOPE | EIA BINDING | | INHIBITION OF IL-2 FUNCTIONAL ASSAYS | | | | RIA[3] | AFFINITY |
|---|---|---|---|---|---|---|---|---|---|---|
| | | AA | | | Neutralization[1] | | | Binding[2] | $^{125}$I-IL-2 | $(M^{-1})$ |
| Group | mAb# | Sequence | H | M | | | | | (%) | |
| | | | | | H | M | R | H | | |
| No.1 – Inhibit human IL-2 function | 5B1 | 1–12 (Thr 3, Lys 8) | + | – | 100 | 0 | 0 | 100 | 100 | $1.2 \times 10^{9}$ |
| | 7B1 | 9–19 (Lys 9, Gln 13, His 16) | + | – | 100 | 0 | 0 | 100 | 100 | $1.7 \times 10^{8}$ |
| | 5A5 | 21–123 | + | – | 100 | 0 | 0 | 91 | 100 | $3.5 \times 10^{7}$ |
| | 13D2 | 42–56 (Try 45, Lys 48, His 55) | + | – | 100 | 0 | 0 | 100 | 100 | $5.2 \times 10^{7}$ |
| No.2 – Do not inhibit IL-2 function | 1A4 | 40–70 (Lys 43, Tyr 45, Lys 48, His 55) | + | – | 0 | ND | ND | 0 | 92 | $1.6 \times 10^{7}$ |
| | 16A4 | 42–56 (Lys 43, Tyr 45, Lys 48) | + | – | 0 | ND | ND | 0 | 80 | $5.9 \times 10^{6}$ |
| | 31A4 | 40–70 (Thr 41, Lys 43, Tyr 45, Lys 48, His 55) | + | + | 0* | 0 | ND | 0* | 100 | $2.8 \times 10^{7}$ |
| | 17A1 | 21–123 | + | – | 0* | 0 | ND | 0* | 100 | $1.1 \times 10^{9}$ |
| | 9A2 | 78–87 | + | – | 0* | 0 | ND | 0* | 100 | $1.0 \times 10^{9}$ |

## TABLE 2 (con't)

### Comparison of the Functional Domains of IL-2 and Epitopes of Monoclonal Anti-IL-2 Antibodies

| MONOCLONAL ANTIBODY | | EPITOPE | EIA BINDING | | INHIBITION OF IL-2 FUNCTIONAL ASSAYS | | | | RIA[3] | AFFINITY |
|---|---|---|---|---|---|---|---|---|---|---|
| Group | mAb# | AA Sequence | H | M | Neutralization[1] | | | Binding[2] | $125_{I-IL-2}$ | $(M^{-1})$ |
| | | | | | H | M | R | H | (%) | |
| No.2 (con't) | 8D1 | 52-70 | + | − | 0 | ND | ND | 0 | 86 | $6.8 \times 10^7$ |
| | 12B5 | 66-87 | + | − | 0 | ND | ND | 0 | 100 | $1.2 \times 10^9$ |
| | 3D5 | 71-87 | + | − | 0 | ND | ND | 0 | 100 | $2.9 \times 10^8$ |
| | 4A4 | 107-121 | + | + | 0* | ND | ND | 0* | 100 | $4.2 \times 10^7$ |
| No.3 – Inhibits mouse and human IL-2 function | 13A6 | 42-56 (Thr 41, Lys 43, Tyr 45, Lys 48) | + | + | 100 | 100 | 30 | 12 | 94 | $3.6 \times 10^7$ |

1. The results are expressed as the percent inhibition of $^3$H-thymidine incorporation in the presence of antibody, compared to control values.

2. The results are expressed as the percent inhibition of $^{125}$I-IL-2 binding in the presence of antibody, compared to control values.

3. The results are expressed as the percentage of total $^{125}$I-IL-2 bound by the antibody, compared to control antibody. Total $^{125}$I-IL-2 input was 20 pmoles.

H, M, R = Neutralization assay performed with either human (H), mouse (M), or rat (R) IL-2.
* Assays performed at a 1000-fold molar excess of antibody to IL-2.
ND = Not determined
AA = Amino Acid

The epitopes of the hybridoma antibodies were identified by EIA reactivity on the synthetic IL-2 peptides described above and by Western blot and dot blot analysis on recombinant IL-2 analogs of Table 1. The Western blot and dot blot assays are described in Example 7.

### Example 7

### Epitope Mapping Analysis

For Western blots using recombinant human IL-2 analogs, an E. coli protein extract is separated by SDS-PAGE, transferred to nitrocellulose and probed with each hybridoma antibody. For dot blots, the E . coli extracts are applied directly to nitrocellulose, dried and probed with each antibody. The antibody bound to the IL-2 analog is visualized with a peroxidase conjugated anti-antibody and the peroxidase substrate, 4-CL-1-napthol. The results are summarized in Table 3 for the 40-70 amino acid region of IL-2. The data shown in Table 3 are a composite of the reactivity of each mAb with different IL-2 analogs as determined by Western and Dot Blot analysis.

TABLE 3

| Micro-Epitope Analysis Within the 40-70 Amino Acid Region of IL-2 | | | | | |
|---|---|---|---|---|---|
| | Monoclonal Antibody Effect on IL-2 Function | | | | |
| | Inhibitory | | Non-Inhibitory | | |
| Peptides Bound | 13D2 | 13A6 | 1A4 | 16A4 | 31A4 |
| 30-70 | + | + | + | + | + |
| 40-70 | + | + | + | + | + |
| 42-56 | + | + | - | + | - |
| 52-70 | - | - | - | - | - |
| IL-2 Analogs Bound | | | | | |
| $Thr^{41} \rightarrow Val$ | + | - | + | + | - |
| $Phe^{42} \rightarrow Tyr$ | + | + | + | + | + |
| $Lys^{43} \rightarrow Glu$ | - | - | - | - | - |
| $Thr^{45} \rightarrow Phe$ | + | - | + | - | - |
| $Met^{46} \rightarrow Leu$ | + | + | + | + | + |
| $Lys^{48} \rightarrow Glu$ | - | - | - | - | - |
| $Lys^{49} \rightarrow Glu$ | + | + | + | + | + |
| $Ala^{50} \rightarrow Trp$ | + | + | + | + | + |
| $His^{55} \rightarrow Asp$ | - | + | - | + | - |
| $Cys^{58} \rightarrow Ser$ | + | + | + | + | + |
| Mouse IL-2 | - | + | - | - | ± |

The monoclonal antibodies of the invention may be used as reagents in conventional affinity chromatography procedures. The monoclonal antibodies of Group 3 are particularly useful as affinity reagents as they may be used to isolate and purify both mouse and human IL-2. The monoclonal antibodies described herein are also useful as reagents in a "Two-Site Sandwich Assay" to measure dilute quantities of IL-2 in biological fluids, e.g. , serum or plasma.

The monoclonal antibodies of the invention are useful in a method for determining the amount of IL-2 protein in a sample of biological fluid, which method comprises the steps of:

(1) binding to a solid carrier a first monoclonal antibody having the ability to bind to an epitope in the IL-2 protein;

(2) contacting a biological fluid sample with said first monoclonal antibody to form an insoluble complex of said peptide and said IL-2 protein in said sample;

(3) contacting the complex of Step 2 with a measured amount of a labelled second monoclonal antibody having the ability to bind an epitope on the IL-2 protein which is different from the epitope bound by said first monoclonal antibody;

(4) separating said solid carrier from said fluid sample and said unreacted labelled antibody; and

(5) measuring the amount of labelled antibody associated with said solid carrier; wherein the amount of said label present on said solid-phase substrate is proportional to the quantity of interleukin-2 protein present in said sample.

As would be recognized by skilled artisans, mAbs from Group 1, 2 and 3 may be used interchangeably in the immunometric assay as long as the mAbs don't bind the same epitope on the IL-2 protein. For example, the Group 3 mAb, 13A6 could not be used with the Group 1, mAb, 13D2 as both mAbs bind to the 41-55 epitope of the IL-2 protein. Preferred for use in the immunometric assay is the Group 1 mAb 5B1 plus the Group 2 mAb 17A1. Both of these mABs have affinities in the range of $1 \times 10^9$ $M^{-1}$.

The Two-Site Sandwich Assay of the invention has been standardized with a sensitivity of 0.5 to 1.0 ng/ml of IL-2 (32.25 to 64.5 fmoles/ml). The linear detecting range of the assay is 0.5-1.0 ng/ml to 10 ng/ml which corresponds to 5-10 U/ml to 100 U/ml. The sensitivity of the assay for IL-2 diluted in buffer containing bovine serum albumin and in 100% human serum is comparable.

As used herein the term "biological fluid" refers to blood, lymph and the like which contains IL-2 protein. The preferred biological fluid for use in the immunometric assay of the intention is serum or plasma, and especially serum.

The first monoclonal antibody of the invention used in the immunometric assay described herein may be immobilized on any of the common supports used in immunometric assays. Among these may be mentioned plastic beads or test tubes made from polyethylene, polystyrene, polypropylene or other suitable material. Also useful are particulate materials such as agarose, cross-linked dextran, and other polysaccharides. The techniques for such bonding are well known to those skilled in the art. For example, antibodies may be bound to polysaccharide polymers using the process described in U.S. Patent No. 3,645,552.

The labelled second monoclonal antibody of the invention may be provided with any of the labels commonly used in prior art immunometric assays. Among these may be mentioned fluorogenic labels for detection by fluorimetry as described in U.S. Pat. No. 3,940,475 and enzymatic markers as described in U.S. Pat No. 3,645,090 or radioisotopic labels ( e.g. , $I^{125}$) using, for example, the procedure of Hunter et al . [Nature 144 :945 (1962)]. An enzymatic label is preferred for use herein.

Example 8

A. IL-2 Two-Site Sandwich Assay

The Group 1 mAb 5B1 was diluted to 20 $\mu$g/ml with Coating Buffer (50 mM Tris-HCl, 150 mM NaCl, pH 8.0) and 100 $\mu$l of the solution was added to each well of a Costar EIA microtiter plate. The plates were incubated overnight at room temperature. Thereafter, the plates were washed three times with Wash Buffer (25 mM sodium phosphate buffer, 150 mM NaCl, 0.05% Tween 20). Prewarmed (37°C) Blocking Buffer (250 $\mu$l) was added to each well of the prewashed plates. The plates were incubated 15-20 min. at 37°C and thereafter washed three times with Wash Buffer. Thereafter, 100 $\mu$l of a serum sample or a standard was added to each well and the plate was incubated overnight at 4°C. The plate was washed 3x with PBS/Tween buffer. To each well was then added 100 $\mu$l of Biotinylated Antibody Diluent (10 or 20 $\mu$g/ml of MAb 17A1) and the plate was incubated 2 hrs at 37°C with shaking. The plate was thereafter washed 3x with PBS/Tween, and 100 $\mu$l of Streptavidin/peroxidase conjugate was added to each well. The plate was then incubated for 15 min. at room temperature when shaking. The plate was washed 3x with PBS/Tween, and 100 $\mu$l of Peroxidase Substrate was added to each well and incubated for 30 min. at room temperature with shaking. The reaction was stopped with 50 $\mu$l of a solution containing 2.5 M $H_2SO_4$ and 50 mM sodium metabisulfite. The plate was read on a Titertek Multiscan MC at 492 nm.

B. Preparation of Standards for Two-Site Sandwich Assay

IL-2 was diluted in 1.4% bovine serum albumin (BSA) or in human serum. The solution was diluted to give a series of dilutions containing from 0.1 ng/ml to 0.1 $\mu$g/ml. Eleven wells were used for the standard curve. One well was used for the buffer blank.

C. Biotinylation of Monoclonal Antibody

The monoclonal antibody from Group 2 identified as 17A1 was dialyzed overnight against 0.1 M sodium bicarbonate buffer (pH 8.4) at 4°C (at least 2 x 2 liters). The protein concentration was adjusted to 1-5 mg/ml with 0.1 M sodium bicarbonate buffer, pH 8.4. There was added 100 $\mu$l of 1.1 mg/ml N-hydroxy succinimide ester of biotin in freshly prepared DMSO to each ml of antibody (1.0 mg). The reaction was allowed to proceed at room temperature for 2 hrs. with occasional shaking. Thereafter, any unreacted ester was blocked by adding 100 $\mu$l of 1 N $NH_4Cl$ and incubating the mixture for an additional 10 mins. Thereafter, 1 ml of 1% BSA in PBS buffer was added for each mg of antibody biotinylated, and the reaction mixture was dialyzed overnight at 4°C against PBS (pH 7.4) containing 0.01% thimerosal (at least 2 liters of PBS were used).

## D. Preparation of Peroxidase Substrate

A solution of o-phenylenediamine (0.4 mg/ml) was prepared in citrate buffer, pH 4.5. To a 10 ml aliquot was added 4 $\mu$l of 30% $H_2O_2$ to give a final concentration of 0.012%.

## E. Composition of Other Buffers and Diluents

1. Coating buffer
50 mM Tris-HCl, pH 8.0
150 mM NaCl
2. Blocking buffer - "FBS buffer + 160 $\mu$g/ml human IgG"
10% FDB (heat inactivated)
1% BSA
0.3% gelatin
25 mM sodium phosphate buffer, pH 8.0
150 mM NaCl
160 $\mu$g/ml human IgG
3. IL-2 and Streptavidin-peroxidase diluent - "1.4% BSA Buffer"
1.4% BSA
0.3% gelatin
25 mM sodium phosphate buffer, pH 7.5
150 mM NaCl
4. Biotinylated Antibody diluent - "FBS Buffer + Tween + huIgG"
FBS buffer + human IgG
0.2% Tween 20
5. O-phenylenediamine buffer
0.1 M citrate buffer, pH 4.5
6. Wash buffer - "PBS/Tween"
25 mM sodium phosphate buffer, pH 7.2
150 mM NaCl
0.05% Tween 20

The above-described Two-Site Sandwich Assay is highly specific and sensitive, because the potential for cross-reactivity has been eliminated or greatly reduced by the use of the monoclonal antibodies of the invention. Since the occurrence of cross-reactivity is reduced or eliminated, the prospect of "false - positive" results is greatly reduced or eliminated.

Comparison of the epitope mapping date with inhibition of IL-2 activity highlights two epitopes which are important for human IL-2 activity: amino acids 1-19 and 41-55. Within the 41-55 area, antibody 13A6 (which neutralizes mouse IL-2 activity) has an epitope which must be different from, but close to, the epitope of 13D2 which does not bind or neutralize mouse IL-2. Evidence that the 1-19 and 40-60 sequences are physically adjacent in the native molecule comes from epitope competition studies. Antibody 5B1, which binds residues 1-12, inhibits a mAb specific for residues 40-70 from binding IL-2, mAbs 17A1 and 3D5 which bind residues 21-123 and 71-87, respectively, are not blocked in the same assay. Of the 75 mAbs analyzed, more than 95% recognize epitopes which are concentrated between amino acids 1-19 and 40-80.

**Claims**

1. A method for the quantitative determination of unglycosylated human IL-2 in the presence of natural glycosylated human IL-2, comprising:

(a) binding a first monoclonal antibody to a solid carrier, which antibody specifically binds to unglycosylated IL-2 but does not substantially bind to glycosylated IL-2;

(b) contacting a biological fluid sample containing human IL-2 with the bound first antibody under conditions in which the antibody specifically binds to unglycosylated IL-2 in the sample to form an insoluble complex of the first antibody and unglycosylated IL-2;

(c) contacting the insoluble complex with a labeled second antibody, which second antibody specifically binds to human IL-2 at an epitope not recognized by the first antibody, under conditions in which the second antibody specifically binds to IL-2 in the complex to form an insoluble complex of the first and second antibodies and unglycosylated IL-2;

(d) separating the complex of step c from unbound antibodies and sample materials; and

(e) measuring the amount of labelled second antibody bound in the complex of step d,

thereby quantifying the amount of unglycosylated human IL-2 in the sample.

2. The method of claim 1 in which the first monoclonal antibody specifically binds to the threonine residue at positions 3 of unglycosylated human IL-2.